# EUROPEAN PATENT APPLICATION

(11) **EP 1 859 754 A1**
(43) Date of publication of application: **28.11.2007**
(21) Application number: 06010669.7
(22) Date of filing: 23.05.2006
(51) Int. Cl.: A61B 19/00

(54) **Apparatus for connecting and manipulating medical instrument tips**

(71) Applicant: Technische Universität München, 80333 München (DE)
(72) Inventor: Jung, Christoph, 85386 Eching (DE); Lauer, Wolfgang, 96049 Bamberg (DE)
(74) Representative: Bosch, Matthias

(57) **Abstract**

An apparatus for connecting and manipulating medical instrument tips is provided that reduces abrasion of driving wires used for manipulating a medical instrument tip and allows a quick and easy exchange of medical instrument tips.
The apparatus comprises a drive portion (1b) for driving at least one pair of driving wires for manipulating a medical instrument tip, a rotation portion (1c), and a connecting portion (1a) for connecting together detachably the medical instrument tip to the drive portion, the connecting portion comprising at least two connecting means (2a,2b) for connecting detachably the at least one pair of driving wires of the drive portion to at least one pair of driving wires of the medical instrument tip, wherein the rotation portion and the connecting portion are adapted to allow a rotation of the medical instrument tip about its principal axis such that the drive portion rotates together with the medical instrument tip, thereby avoiding a twist of the at least one pair of driving wires of the medical instrument tip connected to the at least one pair of driving wires of the drive portion.

## Description

The present invention relates to an apparatus for connecting and manipulating medical instrument tips.

Minimal invasive operative techniques are well-known and broadly employed, especially in the fields of abdominal surgery and cardiosurgery. Apart from cosmetic benefits for patients, minimal invasive techniques have distinct medical advantages as, according to Zylka-Menhorn, postoperative trauma is mainly affected by the size of the incision(s) necessary for the surgical intervention. In minimal invasive operations, manipulators are increasingly employed. Medical instruments are attached to a manipulator controlled by a surgeon. That way, higher precision can be achieved since movements of the surgeon are scaled and possible tremor of his hands can be compensated. However, major drawbacks of this method is the requirement of a large and complex manipulator system located in an operating room, high initial costs and also high maintenance costs, since instruments have to be replaced and overhauled, respectively, after using them, e.g., for about ten times. One reason for the latter is abrasion of driving wires used for manipulating a medical instrument. The abrasion of the driving wires originates from the construction of prior art systems: A medical instrument tip is attached to a coupling apparatus which solely connects the driving wires of the instrument tip and the driving wires of the manipulator system. The motors for manipulating the instrument tip are located together with the motors for moving the manipulator. Thus, when the whole instrument tip is rotated the driving wires are twisted and tend to rub against each other, if they are moved due to subsequent manipulations of the instrument tip.

Therefore, it is an object of the present invention to provide an apparatus for connecting and manipulating medical instrument tips that reduces abrasion of driving wires used for manipulating a medical instrument tip and allows a quick and easy exchange of medical instrument tips.

This object will be solved by the apparatus according to the present invention comprising a drive portion for driving at least one pair of driving wires for manipulating a medical instrument tip, a rotation portion, and a connecting portion for connecting together detachably the medical instrument tip to the drive portion, the connecting portion comprising at least two connecting means for connecting detachably the at least one pair of driving wires of the drive portion to at least one pair of driving wires of the medical instrument tip, wherein the rotation portion and the connecting portion are adapted to allow a rotation of the medical instrument tip about its principal axis such that the drive portion rotates together with the medical instrument tip, thereby avoiding a twist of the at least one pair of driving wires of the medical instrument tip connected to the at least one pair of driving wires of the drive portion.

Therefore, because of the fact that the driving wires are not twisted when rotating the instrument tip, mechanical wear is significantly decreased as abrasion due to the driving wires rubbing against each other during subsequent manipulations of the instrument tip is substantially eliminated.

Advantageously, each of the at least two connecting means comprises a first pin connected to one of the at least one pair of driving wires of the drive portion and a second pin connected to one of the at least one pair of driving wires of the medical instrument tip, each of the first and the second pins having a bayonet fixing for connecting detachably the first pins to the second pins, respectively.

Advantageously, the connecting portion further comprises a first bearing guiding the first pins of each of the at least two connecting means and a second bearing guiding the second pins of each of the at least two connecting means, the first and the second bearing being supported axially shiftable in a direction parallel to the principal axis of the medical instrument tip.

Advantageously, the apparatus further comprises a casing having at least two guideways and wherein the first and the second bearings each comprise at least two studs. Each guideway of the at least two guideways is adapted for guiding one stud of each of the two bearings during a connection process. The guiding takes place in such a way that the bayonet fixings of the first and the second pin of each of the at least two connecting means are closed and then the distance between the bearings is increased to allow the connected first and second pin of each of the at least two connecting means to be moved freely in a direction parallel to the principal axis of the medical instrument tip.

The aforementioned structural features enable an easy exchange of the instrument tip, thereby allowing the instrument tip being exchanged by only one person, for example, during an operation.

In a preferred embodiment of the present invention, the driving portion comprises at least one driving unit for driving the at least one pair of driving wires, the at least one driving unit being adjustable in the moving direction of the at least one pair of driving wires for the purpose of eliminating possible slackness of the at least one pair of driving wires.

Advantageously, the at least one driving unit is adjustable in the moving direction of the at least one pair of driving wires by means of a set screw and a spring.

Thus, a high-precision level vital for manipulating a medical instrument tip is immediately reestablished by adjusting the set crew after changing the instrument tip.

The present invention will be described in more detail by means of a preferred embodiment. The drawings show:
- Figure 1: a general view of a preferred embodiment of the present invention mounted on a supporting structure;
- Figure 2: an illustration of the coupling portion and part of the casing of the embodiment shown in Figure 1;
- Figure 3: an exploded view of the coupling portion of the embodiment shown in Figure 1;
- Figure 4: an exploded view of the driving portion of the embodiment shown in Figure 1;
- Figure 5: a further illustration of the embodiment shown in Figure 1;
- Figure 6: a part of the casing of the embodiments shown in Figure 1.

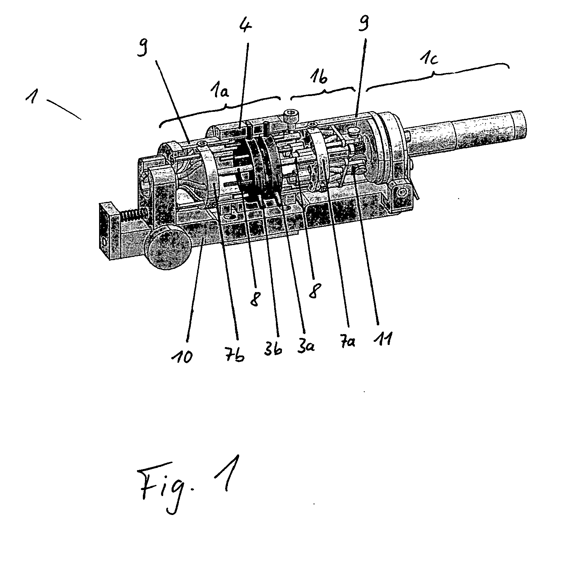

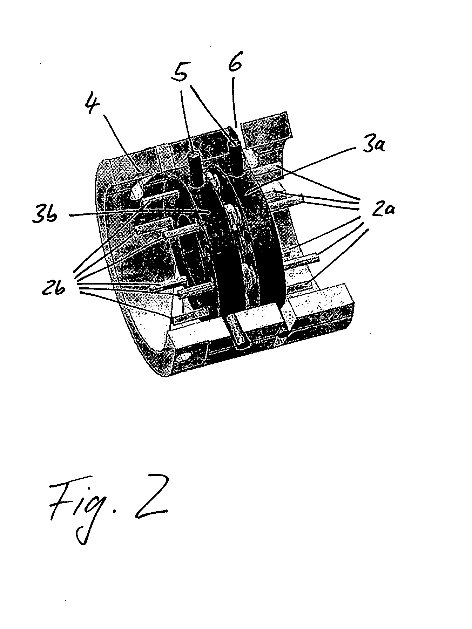

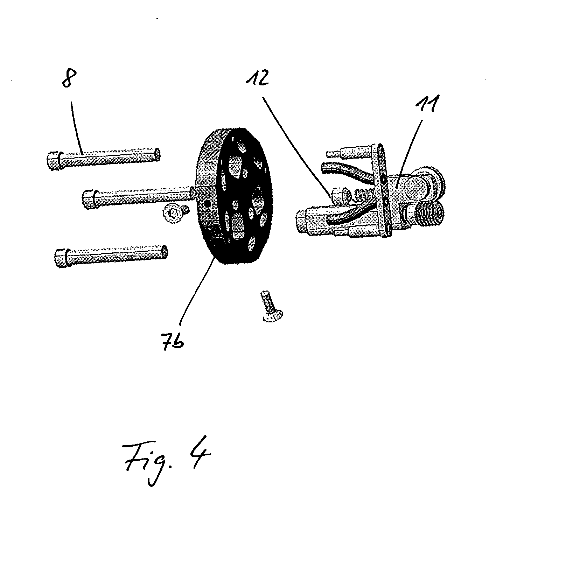

Figure 1 shows a general view of a preferred embodiment of the present invention mounted on a supporting structure 10. Casing 4 and enclosure 9 are depicted only partially to allow a view to the apparatus within the casing 4. There are roughly indicated connecting portion 1 a, driving portion 1 b, and rotation portion 1 c. Connecting portion 1 a and drive portion 1 b will be described in detail with reference to Figures 2 and 4, respectively. Rotation portion 1 c includes a motor and a gear unit for causing a rotation of the apparatus as a whole. Supporting structure 10 supports the apparatus while allowing the apparatus to rotate.

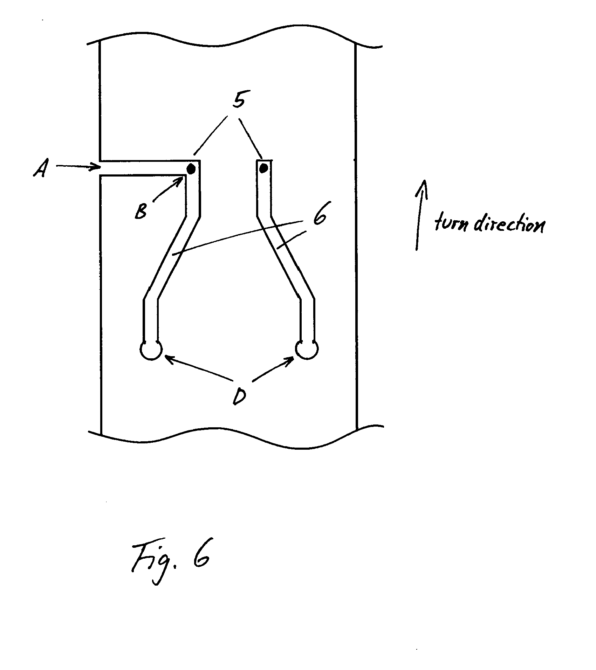

Figure 2 shows the connecting portion 1 a and part of the casing 4 of the embodiment shown in Figure 1. The embodiment shown comprises six connection means (2a, 2b) each including a first 2a and a second pin 2b. Each first pin 2a is connected to the corresponding second pin 2b by means of a bayonet fixing, respectively. The first pins 2a are guided by first bearing 3a and the second pins 2b are guided by second bearing 3b. All pins have non rotation-invariant cross sections inhibiting a rotation within the bearings 3a, 3b having a corresponding hole. Both bearings 3a, 3b further incorporate, for example, three studs 5 in this embodiment. Each pair of studs 5 comprised of one stud 5 of the first bearing 3a and one respective stud 5 of the second bearing 3b is guided by a corresponding guideway 6 of the casing 4 . The guideways facilitate connecting and disconnecting operations which will be described in detail below with reference to Figure 6. Referring again to Figure 1, two supports 7a, 7b are shown, the first one 7a supporting driving units 11 and the second one supporting bowden cables containing the driving wires. The two supports 7a, 7b incorporate supporting pins 8 for the bearings 3a, 3b being axially shiftable in a direction parallel to the principal axis of the medical instrument tip.

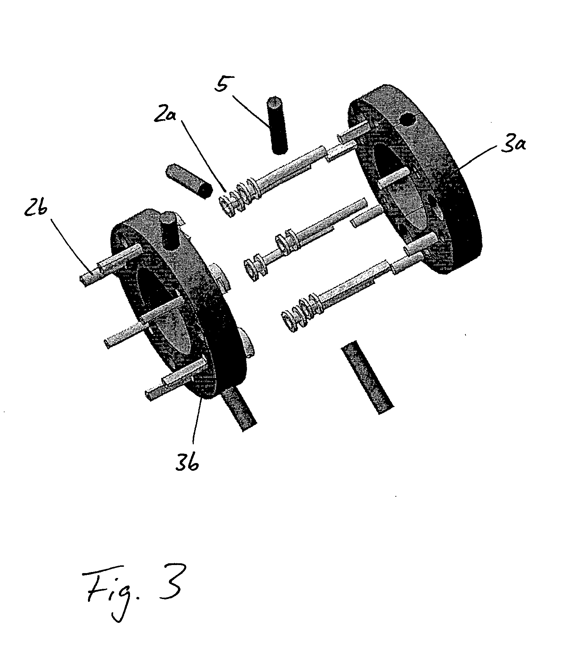

Figure 3 shows an exploded view of the connecting portion of the embodiment shown in Figure 1.

Figure 4 shows an exploded view of the driving portion of the embodiment shown in Figure 1 with, for the sake of clarity, only one driving unit 11 of the three driving units comprised in the embodiment of Figure 1. One driving unit 11 drives one pair of driving wires configured to provide one degree of freedom, i.e. mode of movement, for manipulation the instrument tip connected to the embodiment of the present invention. Set crews and springs 12 are provided for adjusting the driving unit 11 in the moving direction of the corresponding pair of driving wires for the purpose of eliminating possible slackness of the pair of driving wires. Further, the aforementioned support 7a and supporting pins 8 for the bearing 3a are also depicted in Figure 4.

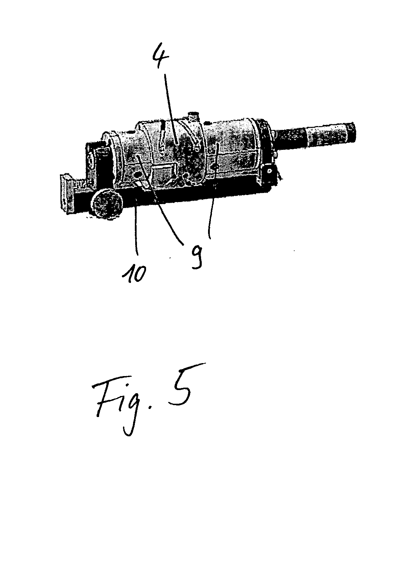

Figure 5 shows an illustration of the embodiment shown in Figure 1 wherein enclosure 9 and casing 4 are fully depicted. Enclosure 9 surrounds the connecting portion 1a and the drive portion 1b protecting them from environmental influences. Enclosure 9 includes two parts detachably connected by a bayonet fixing providing for a strong assembly of the component of the connecting portion 1 a coupled to the instrument tip and the component of the connecting portion 1 a coupled to the driving portion 1 b.

Figure 6 shows, by way of example, a part of the casing 4 of the embodiment shown in Figure 1 comprising one guideway 6 for guiding the studs 5 of the bearings 3a, 3b during connection and disconnection processes. The component of the connecting portion 1 a coupled to the instrument tip (hereinafter referred to as "I-component") is mounted on the component of the connecting portion 1 a coupled to the driving portion 1 b (hereinafter referred to as "D-component") by inserting each stud 5 of the 1-component at point A of the respective guideway of casing 4, which is connected to the D-component. At point B, the connecting means 2a, 2b are aligned and, by turning only the 1-component relative to the casing and the D-component to point C, the first 2a and second pins 2b of the connecting means 2a, 2b are connected closing the bayonet fixings of the first 2a and second pins 2b. By turning the casing 4 in the direction indicated in Figure 6, the distance between the bearings is increased to allow the connected first and second pins 2a, 2b to be moved freely in a direction parallel to the principal axis of the instrument tip. At point D, the studs of both the I-component and the D-component snap in.

## Claims

1. Apparatus comprising a drive portion (1 b) for driving at least one pair of driving wires for manipulating a medical instrument tip, a rotation portion (1c), and a connecting portion (1 a) for connecting detachably the medical instrument tip to the drive portion (1 b), the connecting portion (1 a) comprising at least two connecting means (2a, 2b) for connecting detachably the at least one pair of driving wires of the drive portion (1 b) to at least one pair of driving wires of the medical instrument tip,
**characterized in that**
the rotation portion (1 c) and the connecting portion (1a) are adapted to allow a rotation of the medical instrument tip about its principal axis such that the drive portion (1 b) rotates together with the medical instrument tip, thereby avoiding a twist of the at least one pair of driving wires of the medical instrument tip connected to the at least one pair of driving wires of the drive portion (1 b).

2. The apparatus according to claim 1, wherein each of the at least two connecting means (2a, 2b) comprises a first pin (2a) connected to one of the at least one pair of driving wires of the drive portion (1 b) and a second pin (2b) connected to one of the at least one pair of driving wires of the medical instrument tip, each of the first (2a) and the second pins (2b) having a bayonet fixing for connecting detachably the first pins (2a) to the second pins (2b), respectively.

3. The apparatus according to claim 2, wherein the connecting portion (1 a) further comprises a first bearing (3a) guiding the first pins (2a) of each of the at least two connecting means (2a, 2b) and a second bearing (3b) guiding the second pins (2b) of each of the at least two connecting means (2a, 2b), the first (3a) and the second bearing (3b) being supported axially shiftable in a direction parallel to the principal axis of the medical instrument tip.

4. The apparatus according to claim 3 further comprising a casing having at least two guideways and wherein the first (3a) and the second bearing (3b) each comprise at least two studs (5) and each guideway (6) of the at least two guideways (6) is adapted for guiding one stud (5) of each of the two bearings (3a, 3b) during a connection process in such a way that the bayonet fixings of the first (2a) and the second pin (2b) of each of the at least two connecting means (2a, 2b) are closed and then the distance between the two bearings (3a, 3b) is increased to allow the connected first (2a) and second pin (2b) of each of the at least two connecting means (2a, 2b) to be moved freely in a direction parallel to the principal axis of the medical instrument tip.

5. The apparatus according to one of the preceding claims, wherein the drive portion (1 b) comprises at least one driving unit (11) for driving the at least one pair of driving wires, the at least one driving unit (11) being adjustable in the moving direction of the at least one pair of driving wires for the purpose of eliminating possible slackness of the at least one pair of driving wires.

6. The apparatus according to claim 5, wherein the at least one driving unit (11) is adjustable in the moving direction of the at least one pair of driving wires by means of a set screw and a spring (12).
